# EUROPÄISCHE PATENTSCHRIFT

(11) **EP 3 723 588 B1**
(45) Veröffentlichungstag und Bekanntmachung des Hinweises auf die Patenterteilung: **07.02.2024**
(21) Anmeldenummer: 18830746.6
(22) Anmeldetag: 12.12.2018
(51) Int. Cl.: A61B 5/00

(54) **KOMBINIERTE UNTERSUCHUNG MIT BILDGEBUNG UND LASERMESSUNG**
COMBINED EXAMINATION WITH IMAGING AND A LASER MEASUREMENT
EXAMEN COMBINÉ COMPRENANT IMAGERIE ET MESURE LASER

(30) Priorität: 13.12.2017 DE 102017129837
(43) Veröffentlichungstag der Anmeldung: 21.10.2020
(73) Patentinhaber: Leibniz-Institut für Photonische Technologien e.V., 07745 Jena (DE)
(72) Erfinder: SCHIE, Iwan W., 07745 Jena (DE); YANG, Wei, 07745 Jena (DE); POPP, Jürgen, 07751 Jena (DE)
(74) Vertreter: Maiwald GmbH
(86) Internationale Anmeldenummer: PCT/EP2018/084493
(87) Internationale Veröffentlichungsnummer: WO 2019/115589

(56) Entgegenhaltungen:
- WO-A1-2013/010910
- US-A1- 2007 161 906
- US-A1- 2009 244 260
- US-A1- 2012 123 205
- US-A1- 2017 160 201

## Beschreibung

Die Erfindung betrifft eine Vorrichtung, die es ermöglicht, großflächig durch Bildgebung gewonnene Information von einer Probe mit lokal abgefragter Information über bestimmte Eigenschaften der Probe zu fusionieren.

### Stand der Technik

Zur Untersuchung der Eigenschaften von Proben, wie etwa biologischem Gewebe, werden vielfach bildgebende Verfahren eingesetzt. Insbesondere optische Bilder sind auch an räumlich ausgedehnten Proben, wie etwa einem kompletten Organ, sehr schnell gewinnbar. Beispielsweise kann ein solches Bild die Information enthalten, welcher Anteil des von einer Lichtquelle angebotenen Spektrums von der Probe zurückgeworfen wird.

Mitunter reicht diese Information nicht für eine schlüssige Beantwortung der zu Beginn der Untersuchung gestellten Frage aus. So kann etwa eine optische Veränderung von Gewebe von einer Erkrankung herrühren, aber auch eine andere Ursache haben. Eine chemische Untersuchung, beispielsweise mit molekülspezifischer Raman-Spektroskopie, kann hier Klarheit bringen, ob beispielsweise Tumormarker im Gewebe vorhanden sind.

Eine solche Untersuchung ist jedoch schwierig in-vivo durchführbar. Die meisten Geräte für eine Untersuchung mittels Raman-Spektroskopie sind für die Analyse kleiner Proben auf Objektträgern oder in Küvetten ausgelegt, die einem ausgedehnten Organ zunächst entnommen werden müssten. Beispiele für solche Geräte sind um ein Raman-Spektrometer erweiterte optische Mikroskope. Prinzipiell könnte der Laserstrahl aus einer größeren Entfernung auf das Organ gerichtet werden. Es ist dann jedoch schwierig, genug von dem in alle Richtungen emittierten Raman-gestreuten Licht einzusammeln.

US 2012/123 205 A1 offenbart ein System, mit dem während einer Operation mit Hilfe einer beispielsweise endoskopischen Sonde ein mit sichtbarem Licht angefertigtes Übersichtsbild eines Operationsgebiets angefertigt werden kann, wobei diesem Übersichtsbild dann Informationen überlagert sind, die durch lokale Abfrage von Teilen des Operationsgebiets mit einem aus der Sonde austretenden Laserstrahl erfasst wurden.

US 2017/160 201 A1 offenbart ein weiteres Untersuchungsgerät, mit dem ein ausgedehntes Objekt untersucht werden kann, wobei ein mit sichtbarem Licht angefertigtes Übersichtsbild und lokal mit einem Laserstrahl abgefragte Informationen miteinander fusioniert und synthetisiert werden.

US 2009/244 260 A1 offenbart ein Endoskopsystem, das durch räumliches Scannen und Intensitätsmodulieren eines Laserstrahls ein Punktmuster im untersuchten Bereich erzeugt und aus einem optischen Bild dieses Punktmusters dreidimensionale Formen rekonstruiert.

US 2007/161 906 A1 offenbart ein Gerät zur Unterstützung dermatologischer Eingriffe, das in der Haut verborgene Blutgefäße optisch detektiert und auf die Hautoberfläche projiziert, um beispielsweise eine Behandlung dieser Blutgefäße zu erleichtern.

WO 2013/010 910 A1 offenbart ein Verfahren, mit dem sich bei einem 3D-Scan eines festen Objekts, wie etwa eines Zahns, Störungen durch vorübergehend im Sichtfeld befindliche bewegliche Objekte, wie etwa Wangen oder eine Zunge, herauskorrigieren lassen.

### Aufgabe und Lösung

Es ist daher die Aufgabe der Erfindung, eine Vorrichtung zur Verfügung zu stellen, mit der an einer räumlich ausgedehnten Probe eine Eigenschaft lokal mit einem Laserstrahl abgefragt und diese Eigenschaft bestmöglich für in Bezug auf die abgefragte Eigenschaft räumlich selektive Arbeit an der Probe, wie beispielsweise chirurgische Eingriffe, nutzbar gemacht werden kann.

Diese Aufgabe wird erfindungsgemäß gelöst durch eine Vorrichtung gemäß Anspruch 1. Weitere vorteilhafte Ausgestaltungen ergeben sich aus den darauf rückbezogenen Unteransprüchen.

### Gegenstand der Erfindung

Im Rahmen der Erfindung wurde eine Vorrichtung zur Untersuchung einer Probe entwickelt. Diese Vorrichtung umfasst eine bildgebende Einrichtung zur Gewinnung eines Übersichtsbildes der Probe, wie beispielsweise eine Kamera oder eine Anordnung von Kameras. Die Vorrichtung umfasst weiterhin ein Messinstrument zur lokalen Abfrage mindestens einer Eigenschaft der Probe mit einem Laserstrahl, welcher aus einer Apertur austritt, Verfolgungsmittel zur Ermittlung des Ortes auf der Probe, der aktuell mit dem Laserstrahl abgefragt wird, sowie einen Speicher, in dem die mit dem Laserstrahl abgefragte Eigenschaft mit dem ermittelten Ort auf der Probe assoziiert wird.

Erfindungsgemäß sind die besagten Verfolgungsmittel dazu ausgebildet, den Ort, an dem der Laserstrahl auf die Probe trifft, durch Auswertung des dabei entstehenden Laserpunkts aus dem Übersichtsbild zu ermitteln, und/oder diesen Ort durch Messung von Position und Orientierung der Apertur zu ermitteln.

Es wurde erkannt, dass es auf diese Weise möglich wird, auch bei einer räumlich ausgedehnten Probe, wie etwa einem kompletten Organ, mit der Apertur nah an die Probe heranzukommen. Somit kann die Apertur einen großen Teil des Raumwinkels abdecken, in den die Probe auf die Abfrage mit dem Laserstrahl hin ihrerseits Licht emittiert. Es kann dann ein großer Teil dieses Lichts eingesammelt und der Auswertung zugeführt werden.

Dies bedeutet einen Paradigmenwechsel gegenüber den besagten Mikroskopen, die um Raman-Spektrometer erweitert sind. Bei diesen Mikroskopen ist der Ort auf der Probe, der mit dem Laserstrahl untersucht wird, vorab bekannt, da die Probe beispielsweise auf einem Positioniertisch befestigt ist, der in definierter Weise relativ zu der Apertur bewegt wird. Dadurch ist es in keiner Weise schwierig, die mit dem Laserstrahl abgefragte Eigenschaft einem bestimmten Ort auf der Probe zuzuordnen. Es wurde erkannt, dass es bei räumlich ausgedehnten Proben technisch schwierig ist, die Apertur in vorab bekannter Weise aus großer Entfernung nah an die Probe zu führen. Stattdessen ist es vorteilhaft, die Kenntnis, welcher Ort auf der Probe aktuell mit dem Laserstrahl abgefragt wird, zunächst komplett aufzugeben und nachträglich wieder zu ermitteln.

Der Begriff der Abfrage einer Eigenschaft auf der Probe mit dem Laserstrahl ist jedoch nicht darauf beschränkt, dass die Probe in Antwort auf den Laserstrahl ein optisches Signal erzeugt und dieses ausgewertet wird. Die Probe kann beispielsweise auch durch den Laserstrahl lokal erwärmt und diese Erwärmung im Fernfeld mit einer Wärmebildkamera beobachtet werden. Ebenso kann beispielsweise lokal Material von der Probe abgetragen und in ein Massenspektrometer eingesaugt werden, um die chemische Zusammensetzung zu bestimmen.

Gemäß der Erfindung ist die Apertur Teil einer Sonde, die vom Bediener der Vorrichtung manuell zur Probe führbar ist. Während es üblicherweise gerade angestrebt wird, möglichst viele Schritte zu automatisieren und zu mechanisieren, ist eine manuelle Positionierung der Apertur insbesondere bei der Untersuchung von Gewebe während einer Operation vorteilhaft. Eine mechanisierte Positionierung mit einem Roboterarm würde hier einen erheblichen Aufwand erfordern, wobei insbesondere auch die Sicherheit gewährleistet werden müsste, dass keine Verletzungen durch zu große Kraftwirkung des Roboterarms entstehen. Auch wäre für den Roboterarm viel Platz erforderlich, der in einem Operationsszenario häufig nicht zur Verfügung steht. Indem nun die Positionierung manuell durchgeführt und die Automatisierung auf die nachträgliche Ermittlung des untersuchten Ortes auf der Probe verlagert wird, werden die Stärken des Bedieners einerseits und der Technik andererseits optimal miteinander kombiniert. Dies ermöglicht auch die Realisierung einer transportablen Einheit, die nicht an eine bestimmte Räumlichkeit gebunden ist.

Der Begriff der manuellen Führbarkeit ist nicht darauf beschränkt, dass die Sonde in der Hand gehalten wird. Vielmehr umfasst dieser Begriff auch beispielsweise, dass die Sonde durch den Arbeitskanal eines Endoskops zu einem Organ geführt wird. Im Allgemeinen wird aus Zeitgründen nicht die komplette ausgedehnte Probe mit dem Laserstrahl im Detail untersucht, sondern es werden anhand des Übersichtsbildes bestimmte Orte für diese Untersuchung ausgewählt. Der Bediener kann hier die schnelle Hand-Auge-Koordination nutzen und zugleich auch seinen Tastsinn nutzen, um Verletzungen durch zu große Kraftwirkung auf Gewebe zu vermeiden. Der Bediener kann sich also rein auf die medizinischen Aspekte der Untersuchung konzentrieren, während sich die Vorrichtung im Hintergrund um die Komplexität kümmert, das Ergebnis der Detailuntersuchung durch den Laserstrahl mit dem Übersichtsbild zusammenzuführen.

Vorteilhaft weist die Sonde einen manuell betätigbaren Auslöser für die Abfrage der Eigenschaft durch das Messinstrument auf. Die Abfrage der Eigenschaft ist nicht für jede Untersuchungsart in Echtzeit möglich. Beispielsweise kann die Aufnahme eines Raman-Spektrums einige Sekunden dauern. Dies könnte dazu führen, dass nach dem Positionieren der Sonde an einer interessierenden Stelle zunächst das Ende der laufenden Aufnahme abgewartet werden muss. Wenn die Aufnahme hingegen durch Betätigung des Auslösers gestartet wird, entfällt diese Wartezeit.

Wenn sich der Abstand zwischen der Sonde und der Probe ändert, kann sich auch die Fokusebene des aus der Apertur austretenden Laserstrahls ändern. Inwieweit sich dies auf die Abfrage der Eigenschaft der Probe mit dem Laserstrahl auswirkt, hängt davon ab, welcher physikalische Kontrastmechanismus bei der Abfrage verwendet wird und wie schnell diese Abfrage erfolgt. Beispielsweise kann die Aufnahme eines Raman-Spektrums einige Sekunden in Anspruch nehmen und bei zu starker Relativbewegung zwischen Sonde und Probe "verwackelt" werden, ähnlich wie ein Foto, das mit entsprechend langer Belichtungszeit aufgenommen wird. Die Relativbewegung kann beispielsweise durch eine manuelle Führung der Sonde verursacht werden, aber auch beispielsweise durch die natürliche Bewegung der Probe, bei der es sich etwa um ein lebendes Organ handeln kann. Daher sind in einer weiteren besonders vorteilhaften Ausgestaltung der Erfindung Nachführmittel vorgesehen, die dazu ausgebildet sind, die Fokusebene des aus der Apertur austretenden Laserstrahls einer Änderung des Abstands zwischen der Apertur und der Probe nachzuführen.

Diese Nachführmittel können beispielsweise dazu ausgebildet sein, die Fokusebene des Lasers, und/oder die Fokusebene der Lichteinkopplung in einen zu der Apertur führenden Lichtwellenleiter, automatisch nachzujustieren. Zu diesem Zweck kann der Abstand zwischen der Sonde und der Probe auf beliebige Weise ermittelt werden. Beispielsweise kann die Sonde eine Messvorrichtung für den Abstand zwischen der Sonde und der Probe aufweisen. Diese Messvorrichtung kann beispielsweise einen Sender für eine elektromagnetische Welle, und/oder für Ultraschall, sowie einen Empfänger für die von der Probe reflektierte Welle, bzw. den von der Probe reflektierten Ultraschall, aufweisen. Der Abstand zwischen der Apertur und der Probe kann aber auch beispielsweise anhand eines beliebigen Bildes ermittelt werden, das sowohl die Sonde als auch die Probe zeigt. Dieses Bild kann beispielsweise ein Kamerabild sein, aber auch auf beliebige andere Weise, etwa durch Auswertung von Terahertz-Strahlung, erhalten werden.

Die Nachfokussierung kann beispielsweise mit Hilfe von schnellen mechanischen Verfahrmechanismen, mit der Hilfe von Liquid Linsen oder mit einer beliebigen anderen adaptiven Optik durchgeführt werden.

Eine Nachführung der Fokusebene ermöglicht es insbesondere auch, die Fokusebene stabil zu halten, wenn die Apertur, beispielsweise manuell, über einen ausgedehnten Bereich auf der Probe bewegt wird. Dies hat zur Folge, dass die an unterschiedlichen Orten auf der Probe gewonnenen Messwerte für die Eigenschaft der Probe vergleichbarer werden.

In einer besonders vorteilhaften Ausgestaltung der Erfindung umfassen die Verfolgungsmittel eine Bildauswertungslogik, die dazu ausgebildet ist, einen Ort, an dem die Luminanz des Übersichtsbildes einen Schwellwert überschreitet, einen Schwerpunkt der Luminanz des Übersichtsbildes, und/oder einen Ort, an dem ein räumliches Profil der Luminanz des Übersichtsbildes zum Strahlprofil des Laserstrahls passt, als den Ort auf der Probe zu identifizieren, der aktuell mit dem Laserstrahl abgefragt wird. Das Laserlicht ist wesentlich stärker gerichtet als beispielsweise Lampenlicht, mit dem ein optisches Übersichtsbild angefertigt wird, und somit typischerweise in der Luminanz dominant. Eine sichere Identifikation des Ortes, der aktuell mit dem Laserstrahl abgefragt wird, ist also auch dann möglich, wenn Teile der Probe die gleiche Farbe haben wie der Laserstrahl.

In einer weiteren vorteilhaften Ausgestaltung der Erfindung ist ein Modulator zur Intensitätsmodulation des Laserstrahls mit einer Frequenz ω vorgesehen. Weiterhin ist die Einrichtung zur Gewinnung des Übersichtsbildes dahingehend erweitert, dass sie eine zeitliche Abfolge von Übersichtsbildern aufzunehmen vermag. Die Verfolgungsmittel umfassen eine Bildauswertungslogik, die dazu ausgebildet ist, aus der zeitlichen Abfolge von Übersichtsbildern einen Ort, an dem die Luminanz mit der Frequenz ω moduliert ist, als den Ort auf der Probe zu identifizieren, der aktuell mit dem Laserstrahl abgefragt wird. Auf diese Weise kann der aktuell abgefragte Ort auch dann noch identifiziert werden, wenn die verwendete Laserintensität sehr gering ist und die durch den Laserstrahl bewirkte Luminanz hinter der durch andere Lichtquellen verursachten Dominanz zurückbleibt.

In einer weiteren besonders vorteilhaften Ausgestaltung der Erfindung umfassen die Verfolgungsmittel mindestens zwei Laserscanner oder Funksender zur räumlichen Verfolgung der Apertur, bzw. der Sonde. Diese Geräte können beispielsweise in den Ecken eines Operationsaals, wo sie nicht stören, angebracht sein und die Position sowie die Orientierung der Sonde innerhalb des ganzen Operationssaals verfolgen.

In einer weiteren vorteilhaften Ausgestaltung der Erfindung umfasst das Messinstrument zusätzlich eine Scanvorrichtung, die dazu ausgebildet ist, den Austrittswinkel des Laserstrahls aus der Apertur zu verändern. Auf diese Weise kann die punktförmige Untersuchung mit dem Laserstrahl zur Untersuchung eines begrenzten Gebiets auf der Probe erweitert werden.

In einer besonders vorteilhaften Ausgestaltung der Erfindung enthält das Messinstrument ein Raman-Spektrometer zur Abfrage der chemischen Zusammensetzung der Probe. Jedes Molekül hinterlässt im Raman-Spektrum einen charakteristischen Fingerabdruck, so dass beispielsweise auch die Zusammensetzung von Gemischen eindeutig bestimmt werden kann. Insbesondere lässt sich Tumorgewebe über das Vorhandensein von Tumormarkern eindeutig identifizieren. Weiterhin lässt sich das von der Probe Raman-gestreute Licht durch spektrale Filterung von dem für die Abfrage verwendeten Laserstrahl trennen, da es gegenüber dem Laserstrahl wellenlängenverschoben ist.

Auf das Raman-Spektrum können beliebige Korrekturen angewendet werden. Beispielsweise kann ein fluoreszierender Untergrund durch einen Fit mittels Polynomen, EMSC oder einer Least-Squares-Methode abgetrennt werden.

In einer besonders vorteilhaften Ausgestaltung der Erfindung sind ein Anregungslaser einerseits und das Raman-Spektrometer andererseits über einen Faserkoppler, dessen Teilungsverhältnis wellenlängenabhängig ist, mit einer gemeinsamen zur Apertur führenden Glasfaser verbunden. Der Faserkoppler kann zu diesem Zweck beispielsweise einen dichroischen Spiegel enthalten. Auf diese Weise können auch größere Entfernungen von einigen Metern zwischen dem Anregungslaser, dem Spektrometer und der Probe überwunden werden. Der Anregungslaser und das Spektrometer müssen dann beispielsweise im Operationssaal keinen Platz in unmittelbarer Nähe des Operationsfeldes beanspruchen, sondern können an einem Ort untergebracht sein, an dem sie nicht stören. Die Glasfaser mit der Sonde kann auf beliebigem Wege zum Bediener der Vorrichtung geführt sein, beispielsweise von der Decke des Operationssaals herab, um keine Stolperfallen zu erzeugen.

In einer weiteren besonders vorteilhaften Ausgestaltung der Erfindung ist eine Ausgabeeinheit vorgesehen, die dazu ausgebildet ist, eine Darstellung der in dem Speicher abgelegten, mit dem Laserstrahl abgefragten Eigenschaft dem Übersichtsbild der Probe an dem durch den Speicher assoziierten Ort zu überlagern. Auf diese Weise wird das Übersichtsbild in für den Bediener unmittelbar einsichtiger Weise zu einer "Augmented Reality" aufgewertet.

Beispielsweise kann ein Raman-Spektrum dahingehend ausgewertet werden, dass dann nach einem vorgegebenen Kanon von chemischen Substanzen gesucht wird. Beispielsweise kann eine Linearkombination der Raman-Spektren von Substanzen aus diesem Kanon so an das aufgenommene Raman-Spektrum angefittet werden, dass sich eine maximale Übereinstimmung ergibt. Die Koeffizienten der Linearkombination liefern dann eine Aussage über die Mengenverhältnisse, in denen die gesuchten Substanzen an dem abgefragten Ort vorhanden sind. Es kann nun beispielsweise jeder Substanz aus dem Kanon eine Farbe zugeordnet werden, und diese Farben können beispielsweise in der Darstellung mit Intensitäten aufgetragen werden, die durch die Koeffizienten der Linearkombination bestimmt sind.

Dabei ist der Kanon von Substanzen, nach denen gesucht wird, frei wählbar und kann insbesondere vom Bediener dynamisch angepasst werden. So ist es beispielsweise möglich, der Übersichtlichkeit halber bestimmte Substanzen auszublenden.

Gemäß der Erfindung ist ein Projektor vorgesehen, der dazu ausgebildet ist, eine Darstellung der in dem Speicher abgelegten, mit dem Laserstrahl abgefragten Eigenschaft an dem durch den Speicher assoziierten Ort auf die Probe zu projizieren. Damit kann die "Augmented Reality" noch weiter verfeinert werden dahingehend, dass der Bediener den Blick gar nicht mehr zwischen der Probe, etwa dem Organ, und einem Computerbildschirm hin- und herwandern lassen muss. Der Bediener kann beispielsweise die Sonde über einen Bereich des Organs bewegen, dessen chemische Zusammensetzung ihn interessiert, und die mittels Raman-Spektroskopie ermittelte chemische Zusammensetzung unmittelbar auf dem Organ selbst "einzeichnen". Die Projektion ist insbesondere bei längeren Operationen vorteilhaft, da jeder Wechsel des Blicks zwischen dem Organ und einem Computerbildschirm eine Anpassung der Augen auf eine andere Entfernung notwendig macht. Diese Wechsel können mit der Zeit ermüdend wirken.

In einer weiteren besonders vorteilhaften Ausgestaltung der Erfindung umfasst die bildgebende Einrichtung eine Hellfeldkamera. Das aufgenommene Übersichtsbild entspricht dann der normalen Sehweise des Menschen. Es ist also in dieser Hinsicht kein Umgewöhnen erforderlich, wenn der Bediener den Blick zwischen der Probe und einem Computerbildschirm mit dem Übersichtsbild hin- und herbewegt.

In einer weiteren besonders vorteilhaften Ausgestaltung der Erfindung ist die bildgebende Einrichtung zur Aufnahme eines dreidimensionalen Übersichtsbildes der Probe ausgebildet. Ein dreidimensionales Übersichtsbild kann insbesondere verwendet werden, um einen Projektor dahingehend anzusteuern, dass er die Darstellung der abgefragten Eigenschaft an dem korrekten jeweils im Speicher assoziierten Ort auf die Probe projiziert. Die bildgebende Einrichtung kann insbesondere eine Stereokamera und/oder ein Streifenphotometriegerät umfassen.

Beispielsweise kann der lokale Einfallswinkel der Beleuchtung auf die Probe, und somit die lokale Orientierung der Probenoberfläche, durch einen Least-Squares-Fit an Übersichtsbilder ermittelt werden, die unter Beleuchtung aus verschiedenen Richtungen aufgenommen werden. Es wird dann diejenige Orientierung der Probenoberfläche ermittelt, die zu einer Intensitätsverteilung führt, welche zu den tatsächlich beobachteten Intensitätsverteilungen am wenigsten widersprüchlich ist.

In einer weiteren besonders vorteilhaften Ausgestaltung der Erfindung ist eine Umschalteinrichtung vorgesehen, die dazu ausgebildet ist, die Intensität des Laserstrahls zwischen einem ersten, niedrigeren Niveau für die Abfrage der Eigenschaft der Probe und einem zweiten, höheren Niveau für den Abtrag von Material von der Probe, und/oder für die Veränderung von Material der Probe, umzuschalten. Diese Umschalteinrichtung kann beispielsweise ein Shutter oder eine Pockelszelle sein. Die Vorrichtung kann dann zusätzlich dazu verwendet werden, die mit dem Laserstrahl abgefragte Eigenschaft auch zu verändern. Beispielsweise kann ein chemischer Kontaminant oder ein Tumorgewebe abgetragen werden.

Der für den Abtrag, und/oder für die Veränderung, von Material verwendete Laser muss nicht der gleiche sein wie der für die Abfrage verwendete Laser. Die Laserintensität kann auch umgeschaltet werden, indem der Strahlengang von einem zweiten Laser mit höherer Intensität zur Probe freigegeben wird. Beispielsweise kann der für die Abfrage verwendete Laser ein Dauerstrich-Laser sein, während der für den Abtrag, und/oder für die Veränderung, verwendete Laser ultrakurze Pulse mit sehr hoher Intensität emittiert. Derartige Pulse können unmittelbar mit den Elektronenhüllen von Atomen des abzutragenden Materials wechselwirken. Das Material kann dann abgetragen werden, ohne die Umgebung auf der Probe in größerem Umfang zu erwärmen.

Die Umschalteinrichtung kann insbesondere durch einen manuell betätigbaren, an einer manuell führbaren Sonde angebrachten Auslöser ansteuerbar sein. Der Bediener kann dann beispielsweise unter Nutzung der beschriebenen "Augmented Reality" die Sonde als "chemisches Radiergummi" verwenden, um festgestellte unerwünschte Substanzen oder Gewebeveränderungen durch Überstreichen mit der Sonde direkt "auszuradieren".

In diesem Zusammenhang ist anzumerken, dass die Anwendung der Vorrichtung zur Unterstützung von Operationen zwar ein wesentlicher "use case" ist, aber nicht hierauf beschränkt ist (wobei entsprechende Verfahren nicht Teil der beanspruchten Erfindung sind). Beispielsweise kann auch ein aus einer Metall-Legierung oder aus einem Kunststoff-Gemisch bestehendes Bauteil mit einer manuell führbaren Sonde dahingehend untersucht werden, ob die Zusammensetzung der Legierung bzw. des Gemisches über das gesamte Bauteil homogen ist und das Bauteil somit die durch diese Zusammensetzung versprochenen Gebrauchseigenschaften durch und durch besitzt. Ebenso kann beispielsweise eine Schweißnaht oder eine Klebestelle dahingehend untersucht werden, ob die jeweiligen Eigenschaften homogen sind. Bei der Klebestelle können so beispielsweise Bereiche identifiziert werden, in denen der Kleber nicht in seine ausgehärtete Form durchreagiert hat. Als Fehlerquellen kommen hier beispielsweise eine unzureichende Beleuchtung bei einem lichtaktivierten Kleber oder eine unzureichende Durchmischung der Komponenten eines Mehrkomponentenklebers in Betracht.

In einer weiteren besonders vorteilhaften Ausgestaltung der Erfindung ist die Umschalteinrichtung mit dem Messgerät verbunden, so dass sie automatisch ausgelöst wird, wenn die mit dem Laserstrahl abgefragte Eigenschaft eine vorgegebene Bedingung erfüllt. Beispielsweise kann die Umschalteinrichtung automatisch aktiviert werden, wenn an dem Ort, der mit dem Laserstrahl abgefragt wird, eine bestimmte Substanz identifiziert worden ist. Die Umschalteinrichtung kann also durch einen automatischen Auslösemechanismus ansteuerbar sein, welcher durch das Vorhandensein einer bestimmten chemischen Substanz ausgelöst wird. Das Vorhandensein der chemischen Substanz kann beispielsweise durch Raman-Spektroskopie nachgewiesen werden, aber auch beispielsweise durch die Emission von Fluoreszenzlicht in Antwort auf den für die Abfrage verwendeten Laserstrahl. Dies ermöglicht es dem Benutzer, eine chemisch gesteuerte Abtragung durchzuführen.

Eine chemisch gesteuerte Abtragung ist nicht nur im medizinischen Bereich sinnvoll. Sie kann beispielsweise auch im kosmetischen Bereich angewendet werden, um etwa selektiv Tattoo-Farben chemisch umzuwandeln oder aufzuspalten, ohne auf der behandelten Haut Narben zu hinterlassen. Weiterhin können beispielsweise auch Graffiti-Farben selektiv von Oberflächen entfernt werden, die für die Anwendung chemischer Lösungsmittel zu empfindlich sind.

### Spezieller Beschreibungsteil

Nachfolgend wird der Gegenstand der Erfindung anhand von Figuren erläutert, ohne dass der Gegenstand der Erfindung hierdurch beschränkt wird. Es ist gezeigt:
- Figur 1:: Nicht maßstabsgerechte Schemazeichnung eines Ausführungsbeispiels der Vorrichtung 100;
- Figur 2:: Beispielhafte Darstellung der "Augmented Reality" auf einer Ausgabeeinheit 71 (Figur 2a) oder direkt auf der Probe 2 (Figur 2b);
- Figur 3:: Umschaltung 8 zwischen einem Anregungslaser 36 für die Abfrage der Eigenschaft 23 der Probe 2 und einem Ablationslaser 34 für den Materialabtrag von der Probe 2;
- Figur 4:: Überlagerung optischer und chemischer Information am Beispiel zweier Werkstücke 91 und 92, die mit einer Klebefuge 93 verklebt sind.

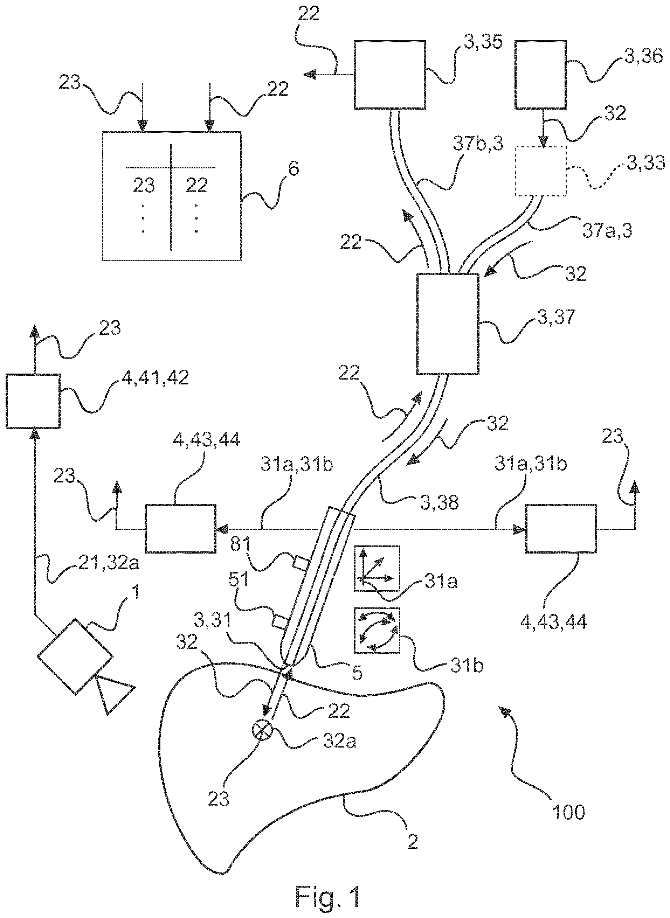

Figur 1 zeigt schematisch ein Ausführungsbeispiel der Vorrichtung 100. Die zu untersuchende Probe 2 ist in diesem Beispiel eine Leber in-vivo. Weitere Organe und Körperteile sind der Übersichtlichkeit halber weggelassen.

Eine Sonde 5 wird von dem Bediener der Vorrichtung 100 über die Probe 2 geführt. Durch die Sonde 5 ist eine Glasfaser 38 geführt, die in einer Apertur 31 mündet. Ein Anregungslaser 36 emittiert einen Laserstrahl 32, der optional in einem Modulator 33 mit einer Frequenz ω moduliert wird. Über eine erste Glasfaser 37a gelangt der Laserstrahl 32 in einen Faserkoppler 37 mit wellenlängenabhängigem Teilungsverhältnis. Der Laserstrahl 32 tritt aus der Apertur 31 aus und erzeugt am Ort 23 auf der Probe 2 einen Laserpunkt 32a. An diesem Ort 23 wird Ramangestreutes Licht erzeugt, das charakteristisch für die lokale chemische Zusammensetzung der Probe 2 als abgefragte Eigenschaft 22 ist. Das Raman-gestreute Licht, symbolisiert durch das Bezugszeichen 22 für die in ihm enthaltene Information, wird durch den Faserkoppler 37 in eine zweite Glasfaser 37b geleitet, die zu einem Raman-Spektrometer 35 führt. Der Abstand zwischen der Apertur 31 und der Probe 2 ist in Figur 1 der Übersichtlichkeit halber stark überhöht gezeichnet. Das Raman-Spektrometer 35 ermittelt die lokale chemische Zusammensetzung 22 der Probe 2 am Ort 23, hat aber selbst noch keine Kenntnis, wo sich dieser Ort 23 auf der Probe 2 befindet.

In Figur 1 sind zwei Möglichkeiten eingezeichnet, mit denen der Ort 23 ermittelt werden kann. Eine diesbezügliche Verfolgungseinrichtung 4 kann mindestens zwei Laserscanner 43 oder Funksender 44 umfassen, die die Position 31a und die Orientierung 31b der Sonde 5, und damit auch der Apertur 31, bestimmen. Die Verfolgungseinrichtung 4 kann alternativ oder in Kombination auch eine Bildauswertungslogik 41, 42 umfassen, die das von einer Kamera 1 gelieferte Übersichtsbild 21 der Probe 2 einschließlich des vom Laserstrahl 32 darin erzeugten Laserpunkts 32a enthält und aus dem Übersichtsbild 21 die Position des Laserpunkts 32a als den Ort 23 auswertet.

Jeder Ort 23 wird zusammen mit der zugehörigen abgefragten Eigenschaft 22 in einem Speicher 6 hinterlegt.

Die Apertur 31, der Anregungslaser 36, der Modulator 33, der Faserkoppler 37, die an den Faserkoppler 37 angeschlossenen Glasfasern 37, 37a und 38 sowie das Raman-Spektrometer 35 bilden zusammen das Messinstrument 3 für die Abfrage der Eigenschaft 22 der Probe 2.

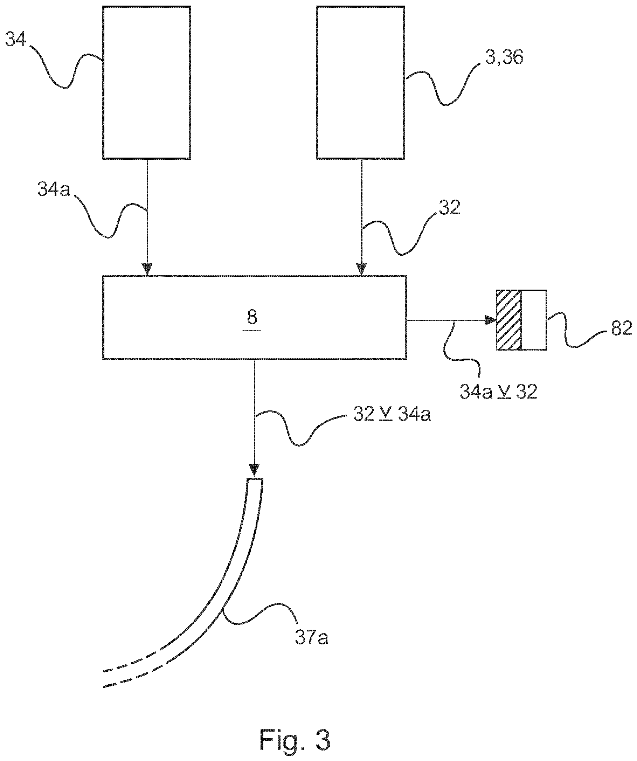

Die Sonde 5 enthält einen ersten Auslöser 51, mit dem durch den Bediener der Vorrichtung 100 die Aufnahme eines Raman-Spektrums durch das Raman-Spektrometer 35 angestoßen werden kann. Ebenso enthält die Sonde 5 einen zweiten Auslöser 81, mit dem die in Figur 3 näher erläuterte Umschalteinrichtung 8 für den Materialabtrag angesteuert werden kann. Die Signalverbindungen der Auslöser 51 und 81 sind in Figur 1 der Übersichtlichkeit halber nicht eingezeichnet.

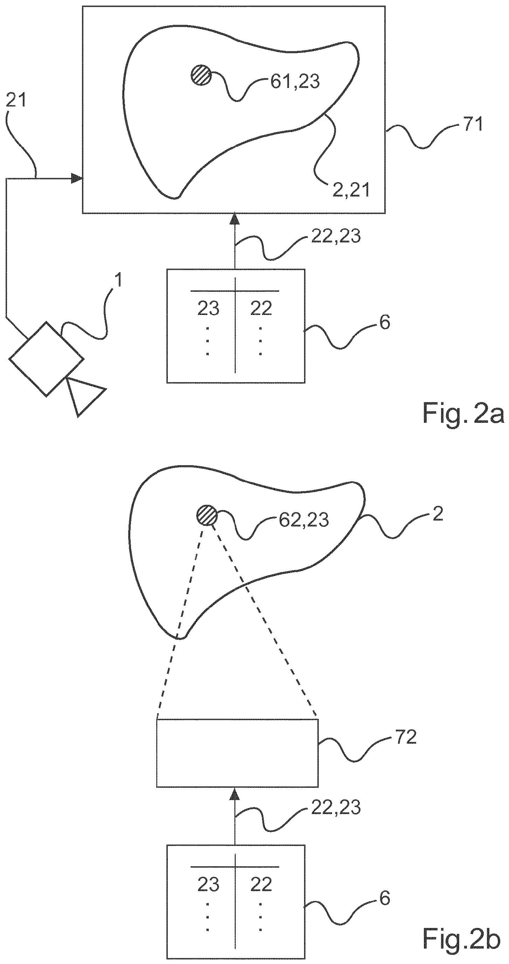

Figur 2a zeigt beispielhaft, wie mit den im Speicher 6 hinterlegten Informationen eine "Augmented Reality" auf einer Ausgabeeinheit 71 dargestellt werden kann. Die Ausgabeeinheit 71 erhält von der Kamera 1 das Übersichtsbild 21 der Probe 2 und zeigt dieses im Hintergrund an. Zugleich erhält die Ausgabeeinheit 71 aus dem Speicher 6 die Werte der abgefragten Eigenschaft 22 zusammen mit den jeweiligen Orten 23 auf der Probe 2. Hieraus bestimmt die Ausgabeeinheit 71 eine Darstellung 61, in der beispielsweise unterschiedliche chemische Substanzen in unterschiedlichen Farben angezeigt werden. Die Darstellung 61 wird dem Übersichtsbild 21 der Probe 2 überlagert.

Figur 2b zeigt beispielhaft, wie eine "Augmented Reality" erzeugt werden kann, die keine Blickwendung weg von der Probe 2 und hin zu einer Ausgabeeinheit 71 erfordert. Ein Projektor 72 erzeugt aus den im Speicher 6 hinterlegten Informationen eine Darstellung 62, die beispielsweise analog zu der Darstellung 61 gemäß Figur 2a farbkodiert sein kann. Im Unterschied zu Figur 2a wird die Darstellung unmittelbar auf die Probe 2 projiziert. Es wird also jeder Wert für die abgefragte Eigenschaft 22 auf den zugehörigen Ort 23 projiziert.

Figur 3 zeigt beispielhaft, wie zwischen einer Abfrage der Eigenschaft 22 und einem Materialabtrag von der Probe 2 umgeschaltet werden kann. Der kontinuierliche Laserstrahl 32 des Anregungslasers 36 und der gepulste Laserstrahl 34a des Ablationslasers 34 sind jeweils in die Umschalteinrichtung 8 geführt. Die Umschalteinrichtung 8 koppelt jeweils genau einen der Strahlen 32 und 34a in die erste Glasfaser 37a ein, die gemäß Figur 1 zum Faserkoppler 37 führt. Der jeweils andere Strahl wird auf ein Beamdump 82 geleitet und dort in Wärme umgewandelt. Auf diese Weise müssen die Laser 36 und 34 selbst nicht ständig an- und ausgeschaltet werden, was schlecht für deren Lebensdauer wäre.

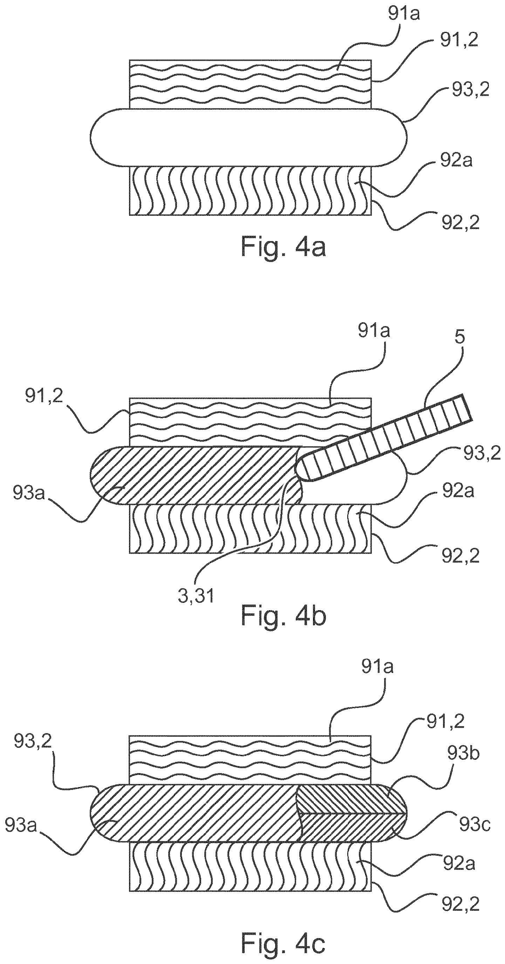

Figur 4 zeigt ein anderes Anwendungsbeispiel, in dem normaler optischer Kontrast und chemischer Kontrast mit Hilfe der Vorrichtung 100 miteinander kombiniert werden können. Als Probe 2 ist eine Anordnung aus einem ersten Werkstück 91 und einem zweiten Werkstück 92, die durch eine Klebefuge 93 miteinander verklebt sind, zu untersuchen.

Figur 4a zeigt diejenigen Merkmale der Probe 2, die in einem normalen optischen Übersichtsbild 21 sichtbar sind. Das erste Werkstück 91 weist im Wesentlichen horizontale Riefen 91a auf, und das zweite Werkstück 92 weist im Wesentlichen vertikale Riefen 92a auf. Die Riefen 91a, 92a sind jeweils bei der Herstellung der Werkstücke 91 und 92 entstanden. Die Klebefuge 93 erscheint farblos und ohne besondere Struktur.

Figur 4b zeigt eine Momentaufnahme, in der die Probe 2 bereits zum Teil mit der Sonde 5 untersucht wurde. Die Klebefuge 93 wird sukzessive von links nach rechts untersucht. Dort, wo die Sonde 5 bereits gewesen ist, wurde identifiziert, dass die Klebefuge 93 aus ordnungsgemäß ausgehärtetem Kleber 93a besteht. Diese Information kann beispielsweise, wie in Figur 2a erläutert, auf einer Ausgabeeinheit 71 ausgegeben werden oder beispielsweise, wie in Figur 2b erläutert, direkt auf die Probe 2 projiziert werden.

Figur 4c zeigt den Zustand, in dem die komplette Klebefuge 93 mit der Sonde 5 abgetastet wurde. In dem in Figur 4b noch nicht untersuchten Bereich der Klebefuge 93 wird nun offenbar, dass dort die erste Komponente 93b und die zweite Komponente 93c des Klebers in getrennten Phasen vorliegen und nicht etwa zur endgültigen Form 93a durchreagiert haben. In diesem Bereich ist die Klebefuge 93 somit fehlerhaft und nicht belastbar.

### Bezugszeichenliste

- 1: Einrichtung zur Gewinnung des Übersichtsbildes 21
- 2: Probe
- 21: Übersichtsbild der Probe 2
- 22: mit Laserstrahl 32 abgefragte Eigenschaft der Probe 2
- 23: Ort auf Probe 2, an dem Laserstrahl 32 Eigenschaft 22 abfragt
- 3: Messinstrument zur lokalen Abfrage der Eigenschaft 22
- 31: Apertur für Laserstrahl 3
- 31a: Position der Apertur 31
- 31b: Orientierung der Apertur 31 im Raum
- 32: Laserstrahl
- 32a: von Laserstrahl 32 auf Probe 2 erzeugter Laserpunkt
- 33: Modulator für Laserstrahl 32
- 34: Ablationslaser
- 34a: Strahl des Ablationslasers 34
- 35: Raman-Spektrometer
- 36: Anregungslaser
- 37: Faserkoppler
- 37a: Eingang des Faserkopplers 37 für Laserstrahlen 32, 34a
- 37b: Ausgang des Faserkopplers 37 für abgefragte Eigenschaft 22
- 38: gemeinsame Glasfaser für Laserstrahl 32, 34a und Eigenschaft 22
- 4: Verfolgungsmittel für Ort 23 auf Probe 2
- 41,42: Bildauswertungslogiken
- 43: Laserscanner
- 44: Funksender
- 5: Sonde
- 51: Auslöser für Abfrage der Eigenschaft 22
- 6: Speicher, der Eigenschaft 22 zu Orten 23 assoziiert
- 61, 62: Darstellungen der Information im Speicher 6
- 71: Ausgabeeinrichtung für Übersichtsbild 21 und Darstellung 61
- 72: Projektor für Darstellung 62 auf Probe 2
- 8: Umschalteinrichtung
- 81: Auslöser für Umschalteinrichtung
- 82: Beamdump
- 91: erstes Werkstück
- 91a: Riefen im ersten Werkstück 91
- 92: zweites Werkstück
- 92a: Riefen im zweiten Werkstück 92
- 93: Klebefuge zwischen Werkstücken 91 und 92
- 93a: vollständig ausgehärteter Kleber
- 93b: erste Komponente des Klebers
- 93c: zweite Komponente des Klebers
- 100: Vorrichtung

## Patentansprüche

1. Vorrichtung (100) zur Untersuchung einer Probe (2), umfassend eine bildgebende Einrichtung (1) zur Gewinnung eines Übersichtsbildes (21) der Probe (2), einen Anregungslaser (36) zur Bereitstellung eines Laserstrahls (32), ein Messinstrument (3) zur lokalen Abfrage mindestens einer Eigenschaft (22) der Probe (2) mit dem Laserstrahl (32), welcher aus einer Apertur (31) austritt, wobei diese Apertur (31) Teil einer Sonde (5) ist, die vom Bediener der Vorrichtung (100) manuell zur Probe (2) führbar ist, weiterhin umfassend Verfolgungsmittel (4) zur Ermittlung des Ortes (23) auf der Probe (2), der aktuell mit dem Laserstrahl (32) abgefragt wird, sowie einen Speicher (6), welcher eingerichtet ist, die mit dem Laserstrahl (32) abgefragte Eigenschaft (22) mit dem ermittelten Ort (23) auf der Probe (2) in dem Speicher (6) zu assoziieren, wobei die Verfolgungsmittel (4) dazu ausgebildet sind, den Ort (23), an dem der Laserstrahl (32) auf die Probe (2) trifft, durch Auswertung des dabei entstehenden Laserpunkts (32a) aus dem Übersichtsbild (21) zu ermitteln, und/oder diesen Ort (23) durch Messung von Position (31a) und Orientierung (31b) der Apertur (31) zu ermitteln, **dadurch gekennzeichnet, dass** ein Projektor (72) vorgesehen ist, der dazu ausgebildet ist, eine Darstellung (62) der in dem Speicher (6) abgelegten, mit dem Laserstrahl (32) abgefragten Eigenschaft (22) an dem durch den Speicher (6) assoziierten Ort (23) auf die Probe (2) zu projizieren.

2. Vorrichtung (100) nach Anspruch 1, **dadurch gekennzeichnet, dass** die Sonde (5) einen manuell betätigbaren Auslöser (51) für die Abfrage der Eigenschaft (22) durch das Messinstrument (3) aufweist.

3. Vorrichtung (100) nach einem der Ansprüche 1 bis 2, **dadurch gekennzeichnet, dass** Nachführmittel vorgesehen sind, die dazu ausgebildet sind, die Fokusebene des aus der Apertur (31) austretenden Laserstrahls (32) einer Änderung des Abstands zwischen der Apertur (31) und der Probe (2) nachzuführen

4. Vorrichtung (100) nach einem der Ansprüche 1 bis 3, **dadurch gekennzeichnet, dass** die Verfolgungsmittel (4) eine Bildauswertungslogik (41) umfassen, die dazu ausgebildet ist, einen Ort, an dem die Luminanz des Übersichtsbildes (21) einen Schwellwert überschreitet, einen Schwerpunkt der Luminanz des Übersichtsbildes (21), und/oder einen Ort, an dem ein räumliches Profil der Luminanz des Übersichtsbildes (21) zum Strahlprofil des Laserstrahls (32) passt, als den Ort (23) auf der Probe (2) zu identifizieren, der aktuell mit dem Laserstrahl (32) abgefragt wird.

5. Vorrichtung (100) nach einem der Ansprüche 1 bis 4, **dadurch gekennzeichnet, dass** ein Modulator (33) zur Intensitätsmodulation des Laserstrahls (32) mit einer Frequenz ω vorgesehen ist, wobei die Einrichtung (1) zur Gewinnung einer zeitlichen Abfolge von Übersichtsbildern (21) ausgebildet ist und wobei die Verfolgungsmittel (4) eine Bildauswertungslogik (42) umfassen, die dazu ausgebildet ist, aus der zeitlichen Abfolge von Übersichtsbildern (21) einen Ort, an dem die Luminanz mit der Frequenz ω moduliert ist, als den Ort (23) auf der Probe (2) zu identifizieren, der aktuell mit dem Laserstrahl (32) abgefragt wird.

6. Vorrichtung (100) nach einem der Ansprüche 1 bis 5, **dadurch gekennzeichnet, dass** die Verfolgungsmittel (4) mindestens zwei Laserscanner (43) oder Funksender (44) zur räumlichen Verfolgung der Apertur (31), bzw. der Sonde (5), umfassen.

7. Vorrichtung (100) nach einem der Ansprüche 1 bis 6, **dadurch gekennzeichnet, dass** das Messinstrument (3) zusätzlich eine Scanvorrichtung aufweist, die dazu ausgebildet ist, den Austrittswinkel des Laserstrahls (32) aus der Apertur (31) zu verändern.

8. Vorrichtung (100) nach einem der Ansprüche 1 bis 7, **dadurch gekennzeichnet, dass** das Messinstrument (3) ein Raman-Spektrometer (35) zur Abfrage der chemischen Zusammensetzung der Probe (2) enthält.

9. Vorrichtung (100) nach Anspruch 8, **dadurch gekennzeichnet, dass** ein Anregungslaser (36) einerseits und das Raman-Spektrometer (35) andererseits über einen Faserkoppler (37), dessen Teilungsverhältnis wellenlängenabhängig ist, mit einer gemeinsamen zur Apertur (31) führenden Glasfaser (38) verbunden sind.

10. Vorrichtung (100) nach einem der Ansprüche 1 bis 9, **dadurch gekennzeichnet, dass** eine Ausgabeeinheit (71) vorgesehen ist, die dazu ausgebildet ist, eine Darstellung (61) der in dem Speicher (6) abgelegten, mit dem Laserstrahl (32) abgefragten Eigenschaft (22) dem Übersichtsbild (21) der Probe (2) an dem durch den Speicher (6) assoziierten Ort (23) zu überlagern.

11. Vorrichtung (100) nach einem der Ansprüche 1 bis 10, **dadurch gekennzeichnet, dass** die bildgebende Einrichtung (1) eine Hellfeldkamera umfasst.

12. Vorrichtung (100) nach einem der Ansprüche 1 bis 11, **dadurch gekennzeichnet, dass** die bildgebende Einrichtung (1) zur Aufnahme eines dreidimensionalen Übersichtsbildes (21) der Probe (2) ausgebildet ist.

13. Vorrichtung (100) nach Anspruch 12, **dadurch gekennzeichnet, dass** die bildgebende Einrichtung (1) eine Stereokamera und/oder ein Streifenphotometriegerät umfasst.

14. Vorrichtung (100) nach einem der Ansprüche 1 bis 13, **dadurch gekennzeichnet, dass** eine Umschalteinrichtung (8) vorgesehen ist, die dazu ausgebildet ist, die Intensität des Laserstrahls (32) zwischen einem ersten, niedrigeren Niveau für die Abfrage der Eigenschaft (22) der Probe (2) und einem zweiten, höheren Niveau für den Abtrag von Material von der Probe (2), und/oder für die Veränderung von Material der Probe (2), umzuschalten.

15. Vorrichtung (100) nach Anspruch 14, **dadurch gekennzeichnet, dass** die Umschalteinrichtung (8) mit dem Messinstrument (3) verbunden ist, so dass sie automatisch ausgelöst wird, wenn die mit dem Laserstrahl (32) abgefragte Eigenschaft (22) eine vorgegebene Bedingung erfüllt.

## Claims

1. An apparatus (100) for examining a sample (2), comprising an imaging device (1) for obtaining an overview image (21) of the sample (2), an excitation laser (36) for providing a laser beam (32), a measuring instrument (3) for locally interrogating at least one property (22) of the sample with the laser beam (32) which emanates from an aperture (31), wherein this aperture (31) is part of a probe (5) that is manually guidable to the sample (2) by an operator of the apparatus (100), further comprising tracking means (4) for determining the location (23) on the sample (2) that is currently being interrogated with the laser beam (32), as well as a memory (6) that is configured to associate the property (22) interrogated with the laser beam (32) with the determined location (23) on the sample (2) in the memory (6), wherein the tracking means (4) are configured to determine the location (23) at which the laser beam (32) impacts the sample (2) by evaluating the so-generated laser spot (32a) from the overview image (21), and/or to determine this location (23) by measuring the position (31a) and orientation (31b) of the aperture, **characterized in that** a projector (72) is provided, said projector (72) being configured to project a rendering (62) of the property (22) interrogated with the laser beam (32) and stored in the memory (6) onto the sample (2) at the location (23) associated by the memory (6).

2. The apparatus (100) of claim 1, **characterized in that** the probe (5) comprises a manually-actuated trigger (51) for the probing of the property (22) by the measuring instrument (3).

3. The apparatus (100) of any one of claims 1 to 2, **characterized in that** adapting means are provided, said adapting means being configured to adapt the focus plane of the laser beam (32) emanating from the aperture (31) to a change of the distance between the aperture (31) and the sample (2).

4. The apparatus (100) of any one of claims 1 to 3, **characterized in that** the tracking means (4) comprise an image processing logic (41), said image processing logic (41) being configured to identify a location at which the luminance of the overview image (21) exceeds a threshold value, a center of gravity of the luminance of the overview image (21), and/or a location at which a spatial profile of the luminance of the overview image (21) matches the beam profile of the laser beam (32), as the location (23) on the sample (2) that is currently being interrogated with the laser beam (32).

5. The apparatus (100) of any one of claims 1 to 4, **characterized in that** a modulator (33) for intensity modulation of the laser beam (32) with a frequency ω is provided, wherein the imaging device (1) is configured for obtaining a temporal sequence of overview images (21), and wherein the tracking means (4) comprise an image processing logic (42) that is configured to determine, from the temporal sequence of overview images (21), a location at which the luminance is modulated with the frequency ω as the location (23) on the sample (2) that is currently being interrogated with the laser beam (32).

6. The apparatus (100) of any one of claims 1 to 5, **characterized in that** the tracking means (4) comprise at least two laser scanners (43) or radio transmitters (44) for spatial tracking of the aperture (31), respectively of the probe (5).

7. The apparatus (100) of any one of claims 1 to 6, **characterized in that** the measuring instrument (3) further comprises a scanning device that is configured to change the exiting angle of the laser beam (32) from the aperture (31).

8. The apparatus (100) of any one of claims 1 to 7, **characterized in that** the measuring instrument (3) comprises a Raman spectrometer (35) for interrogating the chemical composition of the sample (2).

9. The apparatus (100) of claim 8, **characterized in that** an excitation laser (36) on the one hand and the Raman spectrometer (35) on the other hand are connected to a common optical fiber (38) leading to the aperture (31) via a fiber coupler (37) whose splitting ratio is wavelength-dependent.

10. The apparatus (100) of any one of claims 1 to 9, **characterized in that** an output unit (71) is provided, said output unit (71) being configured to superimpose a rendering (61) of the property (22) interrogated with the laser beam (32) and stored in the memory (6) onto the overview image (21) of the sample (2) at a location (23) associated by the memory (6).

11. The apparatus (100) of any one of claims 1 to 10, **characterized in that** the imaging device (1) comprises a bright field camera.

12. The apparatus (100) of any one of claims 1 to 11, **characterized in that** the imaging device (1) is configured for recording a three-dimensional overview image (21) of the sample (2).

13. The apparatus (100) of claim 12, **characterized in that** the imaging device (1) comprises a stereo camera and/or a stripe photometry device.

14. The apparatus (100) of any one of claims 1 to 13, **characterized in that** a switching device (8) is provided, said switching device (8) being configured to switch the intensity of the laser beam (32) between a first, lower level for interrogating the property (22) of the sample (2) and a second, higher level for ablating material from the sample (2), and/or for modifying material of the sample (2).

15. The apparatus (100) of claim 14, **characterized in that** the switching device (8) is connected to the measuring instrument (3), such that it is triggered automatically if the property (22) interrogated with the laser beam (32) fulfils a predetermined condition.

## Revendications

1. Appareil (100) pour examiner un échantillon (2), comprenant un dispositif d'imagerie (1) pour obtenir une image d'ensemble (21) de l'échantillon (2), un laser d'excitation (36) pour fournir un faisceau laser (32), un instrument de mesure (3) pour interroger localement au moins une propriété (22) de l'échantillon avec le faisceau laser (32) qui émane d'une ouverture (31), cette ouverture (31) faisant partie d'une sonde (5) qui peut être guidée manuellement vers l'échantillon (2) par un opérateur de l'appareil (100), dudit appareil (100) comprenant en outre un moyen de suivi (4) pour déterminer l'emplacement (23) sur l'échantillon (2) qui est actuellement interrogé avec le faisceau laser (32), ainsi qu'une mémoire (6) qui est configurée pour associer la propriété (22) interrogée avec le faisceau laser (32) à l'emplacement déterminé (23) sur l'échantillon (2) dans la mémoire (6), les moyens de suivi (4) étant configurés pour déterminer l'emplacement (23) auquel le faisceau laser (32) frappe l'échantillon (2) en évaluant le spot laser ainsi généré (32a) à partir de l'image d'ensemble (21), et/ou pour déterminer cet emplacement (23) en mesurant la position (31a) et l'orientation (31b) de l'ouverture, **caractérisé en ce qu'**un projecteur (72) est prévu, ledit projecteur (72) étant configuré pour projeter un rendu (62) de la propriété (22) interrogée avec le faisceau laser (32) et stockée dans la mémoire (6) sur l'échantillon (2) à l'emplacement (23) associé par la mémoire (6).

2. Appareil (100) selon la revendication 1, **caractérisé en ce que** la sonde (5) comprend un déclencheur (51) actionné manuellement pour le sondage de la propriété (22) par l'instrument de mesure (3).

3. Appareil (100) de l'une quelconque des revendications 1 à 2, **caractérisé en ce que** des moyens d'adaptation sont prévus, lesdits moyens d'adaptation étant configurés pour adapter le plan de focalisation du faisceau laser (32) émanant de l'ouverture (31) à un changement de la distance entre l'ouverture (31) et l'échantillon (2).

4. Appareil (100) de l'une quelconque des revendications 1 à 3, **caractérisé en ce que** les moyens de suivi (4) comprennent une logique de traitement d'image (41), ladite logique de traitement d'image (41) étant configurée pour identifier un emplacement auquel la luminance de l'image d'ensemble (21) dépasse une valeur de seuil, un centre de gravité de la luminance de l'image d'ensemble (21), et/ou un emplacement auquel un profil spatial de la luminance de l'image d'ensemble (21) correspond au profil de faisceau du faisceau laser (32), comme l'emplacement (23) sur l'échantillon (2) qui est actuellement interrogé avec le faisceau laser (32).

5. Appareil (100) de l'une quelconque des revendications 1 à 4, **caractérisé en ce qu'**il est prévu un modulateur (33) pour la modulation d'intensité du faisceau laser (32) avec une fréquence ω, le dispositif d'imagerie (1) étant configuré pour obtenir une séquence temporelle d'images d'ensemble (21), les moyens de suivi (4) comprenant une logique de traitement d'image (42) qui est configurée pour déterminer, à partir de la séquence temporelle d'images d'ensemble (21), un emplacement auquel la luminance est modulée avec la fréquence ω comme l'emplacement (23) sur l'échantillon (2) qui est actuellement interrogé avec le faisceau laser (32).

6. Appareil (100) de l'une quelconque des revendications 1 à 5, **caractérisé en ce que** les moyens de suivi (4) comprennent au moins deux scanners laser (43) ou émetteurs radio (44) pour la poursuite spatiale de l'ouverture (31), respectivement de la sonde (5).

7. Appareil (100) de l'une quelconque des revendications 1 à 6, **caractérisé en ce que** l'instrument de mesure (3) comprend en outre un dispositif de balayage qui est configuré pour modifier l'angle de sortie du faisceau laser (32) de l'ouverture (31).

8. Appareil (100) de l'une quelconque des revendications 1 à 7, **caractérisé en ce que** l'instrument de mesure (3) comprend un spectromètre Raman (35) pour interroger la composition chimique de l'échantillon (2).

9. Appareil (100) selon la revendication 8, **caractérisé en ce qu'**un laser d'excitation (36) d'une part et le spectromètre Raman (35) d'autre part sont connectés à une fibre optique commune (38) menant à l'ouverture (31) par l'intermédiaire d'un coupleur de fibre (37) dont le rapport de division dépend de la longueur d'onde.

10. Appareil (100) de l'une quelconque des revendications 1 à 9, **caractérisé en ce qu'**une unité de sortie (71) est prévue, ladite unité de sortie (71) étant configurée pour superposer un rendu (61) de la propriété (22) interrogée avec le faisceau laser (32) et stockée dans la mémoire (6) sur l'image d'ensemble (21) de l'échantillon (2) à un emplacement (23) associé par la mémoire (6).

11. Appareil (100) de l'une quelconque des revendications 1 à 10, **caractérisé en ce que** le dispositif d'imagerie (1) comprend une caméra à champ lumineux.

12. Appareil (100) de l'une quelconque des revendications 1 à 11, **caractérisé en ce que** le dispositif d'imagerie (1) est configuré pour enregistrer une image d'ensemble tridimensionnelle (21) de l'échantillon (2).

13. Appareil (100) de la revendication 12, **caractérisé en ce que** le dispositif d'imagerie (1) comprend une caméra stéréo et/ou un dispositif de photométrie en bande.

14. Appareil (100) de l'une quelconque des revendications 1 à 13, **caractérisé en ce qu'**un dispositif de commutation (8) est prévu, ledit dispositif de commutation (8) étant configuré pour commuter l'intensité du faisceau laser (32) entre un premier niveau, inférieur, pour interroger la propriété (22) de l'échantillon (2) et un second niveau, supérieur, pour l'ablation de matière de l'échantillon (2), et/ou pour modifier la matière de l'échantillon (2).

15. Appareil (100) selon la revendication 14, **caractérisé en ce que** le dispositif de commutation (8) est connecté à l'instrument de mesure (3), de sorte qu'il est déclenché automatiquement si la propriété (22) interrogée par le faisceau laser (32) remplit une condition prédéterminée.
